# EUROPEAN PATENT APPLICATION

(11) **EP 3 338 746 A1**
(43) Date of publication of application: **27.06.2018**
(21) Application number: 17207645.7
(22) Date of filing: 15.12.2017
(51) Int. Cl.: A61F 13/475

(54) **ABSORBENT ARTICLE FOR FEMININE USE WITH ANTI-DEFORMING ELEMENTS**

(30) Priority: 20.12.2016 MX 2016017374
(71) Applicant: Grupo P.I. Mabe S.A. de C.V., Colonia Loma, Puebla 72230 (MX)
(72) Inventor: SANCHEZ SERRANO, Liliana, 72230 Puebla, Puebla (MX); PINEDA MORENO, Omar, 72230 Puebla, Puebla (MX); CANALES ESPINOSA DELOS MONTEROS, Carlos, 72230 Puebla, Puebla (MX); SANCHEZ FERNANDEZ, Lucia del Carmen, 72230 Puebla, Puebla (MX)
(74) Representative: Larangé, Françoise

(57) **Abstract**

The present invention covers an absorbent article for feminine use (e.g., sanitary towel, pantiliner, light incontinence pad) having a pattern of anti-deforming elements in the top permeable cover of the article with an asymmetrical arrangement in the longitudinal direction and a symmetrical arrangement in the transverse direction; such that a first pair of hook-shaped anti-deforming and longitudinally facing elements are provided at the back region; a second pair of arc-shaped anti-deforming and transversally facing elements are provided at the central region, and an individual half ellipse-shaped anti-deforming element, which ridge faces the central region, is located at the front region of the article, such that the core has a higher strength against deformation and narrowing.

## Description

### Background of the invention

Feminine use absorbent articles serve the function of absorbing and containing the menstrual flow, determined volumes of urine and body fluids, and are characterized by having an absorbent core located between a top permeable cover and a bottom waterproof cover as basic components. After being used for a certain period of time said feminine use absorbent articles are disposed of. Female use absorbent articles include sanitary towels, pantiliners and some female incontinence pads; these articles may have different designs such as: straight, anatomical, winged, wingless, thick, thin, ultra-thin, for night use, for day use, for regular flow, for abundant flow, with channels, with embossements, etc.

The present invention refers to the incorporation of anti--deforming elements with different configurations and orientation, in an absorbent article for feminine use or the like, which increase the strength of the absorbent core against the compressive forces caused by the body movement - , mainly between the legs of the wearer, which causes the absorbent core to be narrowed and deformed in this area, resulting in discomfort and irritation to the wearer, as well as risks of leakages, since the deformation may form unprotected areas by which the body fluids can reach the garment which is in contact with the article.

There are some patents concerning the use of channels or elements that provide resistance against deformation of the absorbent core in these types of articles, such as:
International patent application PCT WO2016068957A1 of Kimberly-Clark Worldwide Inc., discloses an absorbent article with a distribution layer located at the top permeable cover and stiffener elements extending longitudinally in the back portion of the absorbent article to avoid bulging and twisting thereof.

US patent 8,878,000 of Procter & Gamble refers to an absorbent article, for example, sanitary towels or pantiliners, reinforced in the central region by a structure comprised by two transverse channels parallel between each other, with a density from 5 to 500% greater than the density of the central region of the article located between both channels, where the fluids directly reach.

US patent 4,886,513 of Kimberly-Clark Co., refers to an absorbent towel with rigid-flexible elements which reinforce the structure of the article, keeping the shape of the towel during its use. The invention claims the use of a polyethylene flat strip which extends along the periphery of the article and which recovers the original shape of the article after being subjected to some deformation.

Previous patents cover the use of elements (e.g. channels, reliefs, rigid elements and/or flexible elements) added to the sanitary towels aimed to providing strength against forces that may deform the product; referring specifically to the use of channels designed for this purpose, the proposals so far have not proved to be efficient to avoid the deformation of the absorbent core, eliminating the risks of leakages and the discomfort for the wearer. The present invention provides an improvement in the strength against deformation of the central region of an absorbent article for feminine use, due to the implementation of anti-deforming elements, preferably channels, asymmetrically distributed in the longitudinal direction which force the core to remain extended so that at the time of being subjected to different forces of use it is not deformed or narrowed.

Another advantage of the present invention is that some of the anti-deforming elements are geometrically similar to the crotch panels of the feminine garments, so that the article follows the same form of the garment and fits as one single piece, allowing -the moving garment to be accompanied by the article and to stay attached. In this way, the design of the article has enough coverage and strength in the part between the legs of the wearer once the article is placed on the underwear garment to protect her from the menstrual flow, urine and/or body fluids.

In addition, notwithstanding that the anti-deforming elements are not symmetrical throughout the article; this may be placed indistinctly in any direction along the garment, providing the desired fit and comfort, facilitating the wearing thereof for the user.

### Objects of the Invention

The object of the present invention is to avoid the deformation of the absorbent core of an absorbent article for feminine use, such as a sanitary towel, a pantiliner or an article for light female incontinence, as a result of the movement and compressive force caused by the wearer during use, by incorporating anti-deforming elements in the region of the article along the crotch, with different arrangements, longitudinally asymmetrical.

Another object of the present invention is to provide the required protection to the underwear garment of the wearer through the use of anti-deforming elements that perfectly fit in the crotch panel of the underwear garment coupling thereto as one single piece.

A further object of the invention is that the absorbent article may be used with the orientation of the anti-deforming channels in any direction, despite said channels are longitudinally asymmetrical, thus easing the placement of the article in the underwear garment of the user.

### Brief Description of the Drawings

Figure 1 illustrates an absorbent article for feminine use with the anti-deforming elements of the present invention.
Figure 2 is a cut along the longitudinal axis "Y" of Figure 1.
Figure 3 shows the distribution of the anti-deforming elements and the orientation thereof in the article of the invention.
Figures 4, 5 and 6 show three different alternative arrangements of the anti-deforming elements of the present invention.

### Detailed Description of the Invention

The disposable sanitary towels are hygienic articles that serve the function of absorbing and containing the menstrual flow, the same are characterized by having an absorbent core located between a permeable cover and a waterproof cover as basic components. They are disposed of after being used for a determined period of time. In the market, we may find different designs of sanitary towels, such as: thick, thin, ultra-thin, for night use, for regular flow, for abundant flow, winged, wingless, with channels, with embossments, etc.

A pantiliner is a hygienic article used by women usually as a protection against small volumes of body fluids (e.g., vaginal secretions or premenstrual flow), as well as for the prevention of odors and stains on the underwear. The pantiliners are thinner and smaller compared to the feminine sanitary towels, and are generally designed to cover only the crotch portion of the underwear garment of the user (crotch panel), so besides abstracting a lower volume of fluids, they are more discrete.

An incontinence pad is a disposable absorbent article similar to a sanitary towel, but with the absorption required to absorb certain volumes of urine, such that they may be used by women who suffer from slight incontinence. As with the sanitary towels, we can find in the market incontinence pads with different configurations and capacities of absorption.

Hereinafter, the description of the invention shall be focused on pantiliners, nevertheless, the design of the anti-deforming elements herein described may be used in any kind of absorbent article for feminine use.

As seen inFigure 1, the pantiliners (1) have, as basic components, a top permeable cover (3) that allows the entrance of fluids and which will be in contact with the skin of the wearer during use; a waterproof bottom waterproof cover (4) to prevent leakage of fluids, which will be in contact with the underwear garment of the user during use and an absorbent core (5) provided between the top permeable and the bottom waterproof cover.

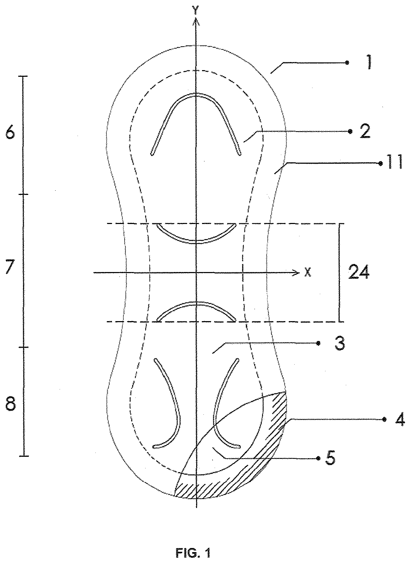

The article has been divided, for illustrative purposes, in three regions in the longitudinal direction, a front region (6), a central region (8) and a back region (7). In the pantiliners, the three regions mentioned above (6,7,8) cover the entire absorbent article as shown in Figure 1, while in the feminine towels, incontinence pads and similar absorbent articles which have a greater size as compared to the pantiliners, the three regions (6,7,8) are located only in the area overlying the crotch panel of the underwear garment, i.e., between the legs of the wearer.

The top (3) and bottom covers (4) of the pantiliner, are longer and wider than the absorbent core (5), the parts protruding thereof comprise the periphery (11) of the article, which is bounded by an edge (12), wherein the top permeable cover (3) and the bottom waterproof cover (4) are attached by means of adhesive, ultrasound, thermal processes, mechanical processes, seams or some other attachment method. Figure 1 shows an oval shape of the periphery (11), however it may be rectangular, hourglass-shaped, or any other form suitable for the purposes that the article may have.

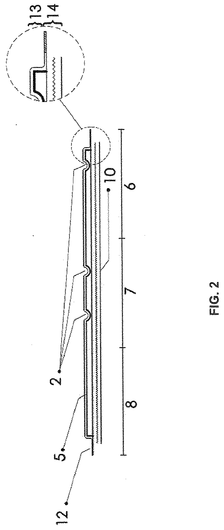

Figure 2 shows a cut of the article of the invention along the longitudinal central axis "Y" of Figure 1. It may be seen that the pantiliner has a sensitive adhesive (9) provided on the outer part of the bottom waterproof cover (5), covered with a silicone material layer (10) that protects the adhesive (9) while it is not used for adhering the pantiliner to the garment of the wearer; Figure 2 illustrates a single rectangular strip of adhesive sensitive (9), however two or more strips or any type of arrangement for this sensitive adhesive (9) may be used.

The feminine absorbent article (1), as seen in Figure 2, has a top portion (13), comprised by the top permeable cover (3), anti-deforming elements (2) and an absorbent core (5), and a bottom portion (14) comprising the bottom waterproofcover (4), the sensitive adhesive (9) and the silicon element (10).

The anti-deforming elements (2) are arranged in the top portion (13) of the article, that is to say, they are disposed on the top permeable cover (3) and comprise 10% to 90% of the thickness of the absorbent core (5).

Hereinafter we will talk about channels (2), although for the present invention, the anti-deforming elements may be reliefs, folds, rigid elements, rigid-flexible elements, continuously or discontinuously embossed channels, or a combination thereof.

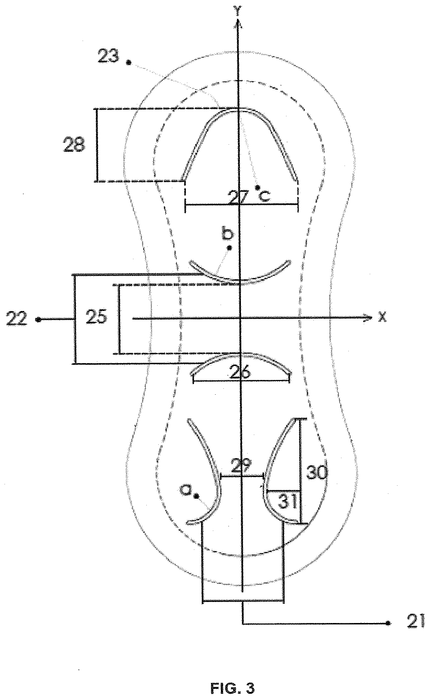

The anti-deforming elements (2) of the present invention, detailed in Figure 3, are comprised by a first pair of embossed channels (21), a second pair of embossed channels (22) and an individual embossed channel (23). The first pair of channels (21), is provided as a mirror, one on each side of the central axis "Y" and located at the back region (8) of the article, longitudinally oriented, with a shape similar to a hook, with the convex region of the hook facing the central axis "Y"; the second pair of channels (22) has an arched shape and is placed in the central region (7) as a mirror about the transverse axis "X", transversely oriented, with the convex region of the arch thereof facing the transverse axis "X"; the individual channel (23) is located in the front region (6) of the pantiliner, is shaped like a half-ellipse whose focal axis matches the longitudinal axis "Y" of the absorbent article, the concave region facing the central region (8) of the absorbent article.

The first pair of channels (21) provided in the back region (8) are hook-shaped channels with a length of at least 3.0 cm, preferably between 2.5 and 10 cm, and even more preferably between 3.0 and 5.0 cm from an imaginary straight line (30) drawn from one end to the opposite end of the channel. The distance defined from the imaginary line to the most distant point of the arc of the hook (31), is between 4 and 12 mm, preferably from 5 to 10 mm. Said pair of channels has a distance between vertex and vertex located in the concavity of the hook (29) from 1 to 5 cm, preferably from 1.2 to 4 cm.

A second pair of channels (22) provided in the central region (7) of the absorbent core are symmetrically arc-shaped with a length of at least 2.0 cm, preferably between 1.5 and 10 cm, and even more preferably between 2.5 and 5.0 cm from an imaginary straight line (26) drawn from one end to another end of the channel. On the other hand, the distance (25) between vertex and vertex of the convex channels in the central region is from 1 to 5 cm, preferably between 1.5 and 4 cm, and even more preferably between 2 and 3 cm. The distance between vertex and vertex of the first pair of channels of the back region of the absorbent article, is less than the distance between vertex and vertex of the second pair of channels located in the central region of the absorbent article. The second pair of channels defines a geometrically region similar to the crotch panel of the garment.

The individual channel (23) provided in the front region (6) of the absorbent article is shaped as a half-ellipse, whose concave face is oriented towards the central region (7) of the absorbent article such that the focal axis thereof matches the longitudinal axis "Y" of the pantiliner. The length of an imaginary straight line (27) drawn from one end to another of the channel must be at least 3.0 cm, preferably from 2.0 to 10.0 cm and even more preferably from 3.0 to 5.0 cm. Furthermore, the distance of the imaginary line (27) to the vertex of the individual channel (28) has a length between 1 to 5 cm, preferably from 2 to 4 cm.

Figure 3 shows the radio of the arcs of each of the channels (2), the first pair of channels (21) provided in the back region (8) are hook-shaped with a convex region having a radius (a) measuring 5-12 mm, preferably between 7-10 mm. The second pair of channels (22) is arc-shaped having a radius (b) measuring between 10 and 35 mm, most preferably between 15 and 25 mm. The individual channel (23) is shaped as a half-ellipse with a focal matching the "Y" axis of absorbent article and which concave region has a radio (c) measuring 5 to 15 mm, preferably 8-12 mm.

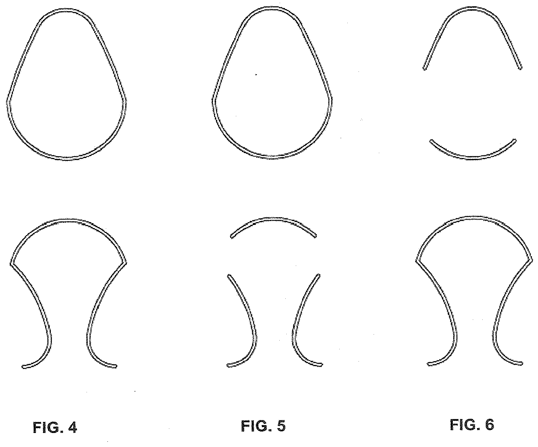

Each of the channels (2,21,22,23), shown in Figure 3 are spaced on the surface of the feminine absorbent article so that they do not cross with each other, however they may or may not form a closed circuit between two or more channels and may form a larger channel , as exemplified in Figures 4, 5 and 6. The article (1) of the invention, has five channels asymmetrically distributed in the longitudinal direction of the absorbent article, however it may have at least three channels (2) or anti-deforming elements.

The absorbent article for feminine use has, in the regions where the channels are provided, a density of 0.10 to 0.20 g/cm³, preferably of 0.12 to 0.16 g/cm³, which is greater than the density of the absorbent core in the areas with no channels.

As mentioned, the anti-deforming elements (2) of the invention are provided in the portion of the article which is located overlying the crotch panel of the underwear garment of the wearer, i.e., in the case of the pantiliners, the anti-deforming elements, as described, are placed throughout the article; in the case of the sanitary towels or the feminine incontinence pads, the anti-deforming elements (2) are placed along the central or crotch portion thereof; in this case, the article may have channels engraved with the desired shape and arrangement, in the areas thereof lacking of anti-deforming elements.
Although the invention has been described in detail in function of the preferred forms of implementation, the scope thereof comprises any kind of change or modification thereto which may be apparent or obvious for a skilled technician.

## Claims

1. An absorbent article for feminine use such as a sanitary towel, a pantiliner or an article for light incontinence, with a front region, a central region and a back region, all of them provided in the crotch area of the article such that they cover the crotch panel of the feminine underwear garment when being placed therein; the article has anti-deforming elements, wherein the anti-deforming elements pattern is focused in the crotch portion of the article and have an asymmetrical arrangement in the longitudinal direction and a symmetrical arrangement in the transverse direction, and are comprised by:
• a first pair of hook-shaped anti-deforming elements in the back region of the article,
• a second pair of arc-shaped anti-deforming elements, provided in the central region of the article,
• an individual half ellipse-shaped anti-deforming element, which focal axis matches the "Y" axis, and is located at the front region of the article.

2. An absorbent article for feminine use as described in claim 1, wherein the first pair of anti-deforming elements provided at the back region of the article has a hook-like shape which convex face is facing the longitudinal axis "Y" and wherein the distance between vertex and vertex of the first pair of convex anti-deforming elements of the back region of the article is from 1 to 5 cm, preferably between 1.2 and 4 cm.

3. An absorbent article according to claim 2, wherein the length of an imaginary straight line from end to end of the hook of the first pair of convex anti-deforming elements provided at the back region of the article is from 1 to 10 cm, preferably from 2 to 5 cm.

4. An absorbent article for feminine use according to claim 2, wherein the distance between the imaginary line drawn from both ends of each hook-shaped channel of the first pair of anti-deforming elements provided as mirror in the back region of the article to the most distant point of the concave region is from 4 to 12 mm and preferably from 5 to 10 mm.

5. An absorbent article for feminine use as described in claim 1, wherein the second pair of anti-deforming elements provided as mirror at the central region has a convex arrangement facing the transverse axis "X" of the article.

6. An absorbent article for feminine use according to claim 5, wherein the distance between vertex and vertex of the second pair of convex anti-deforming elements of the central region of the article is from 1 to 5 cm, preferably between 1.5 and 4 cm and more preferably between 2 and 3 cm.

7. An absorbent article according to claim 5, wherein the length of an imaginary straight line drawn from end to end of the arc of the second pair of convex anti-deforming elements of the central region is from 2 to 5 cm, preferably from 2.5 to 3 cm.

8. An absorbent article for feminine use according to the previous claims, wherein the distance between vertex and vertex of the first pair of anti-deforming elements provided at the back region is smaller than the distance between vertex and vertex of the second pair of anti-deforming elements provided at the central region.

9. An absorbent article for feminine use according to claim 1, wherein the individual half ellipse-shaped anti-deforming element which has the focal axis matching the "Y" axis of the absorbent article and is provided at the front region of the absorbent article, has an imaginary line from end to end of the anti-deforming element whit a length from 2 to 10 cm, preferably from 3 to 6 cm.

10. An absorbent article for feminine use according to claim 9, wherein the distance between the imaginary line drawn from end to end of the individual half ellipse-shaped anti-deforming element and the vertex thereof is between 1 to 4 cm, preferably of 2 to 3 cm.

11. An absorbent article for feminine use according to the previous claims, wherein the anti-deforming elements are channels.

12. An absorbent article according to the previous claims, wherein the regions where the anti-deforming elements are placed have a density of 0.10 to 0.20 g/cm³, preferably of 0.12 to 0.16 g/cm³.

13. An absorbent article according to the previous claims, wherein the anti-deforming elements are provided at the top portion of the article, preferably at the top permeable cover and cover from 10 to 90% of the thickness of the absorbent core.

14. An absorbent article for feminine use according to claims 1 to 10, wherein the anti-deforming elements are reliefs, folds, rigid or rigid-flexible elements.

15. An absorbent article for feminine use according to claims 1 to 10, wherein the anti-deforming elements may be a combination of any of the following elements: channels, reliefs, folds, rigid elements, rigid-flexible elements.

16. An absorbent article for feminine use as described in the previous claims, wherein the anti-deforming elements are spaced such that they do not cross with each other or form a closed circuit.

17. An absorbent article for feminine use as described in claim 15, wherein the channels thereof are placed such that they form a closed circuit.
